# EUROPÄISCHE PATENTANMELDUNG

(11) **EP 0 567 749 A2**
(43) Veröffentlichungstag der Anmeldung: **03.11.1993**
(21) Anmeldenummer: 93104076.0
(22) Anmeldetag: 12.03.1993
(51) Int. Cl.: A61B 17/22

(54) **Instrument für die Lithotripsie**

(30) Priorität: 29.04.1992 DE 4214148
(71) Anmelder: Richard Wolf GmbH, D-75438 Knittlingen (DE)
(72) Erfinder: Bonnet, Ludwig, W-7134 Knittlingen (DE)
(74) Vertreter: Wilcken, Thomas, Dipl.-Ing.

(57) **Zusammenfassung**

Es ist ein Instrument für die Lithotripsie mit einer durch einen Antrieb (11) in Langsschwingungen versetzbaren Sonde (12) beschrieben, die durch einen Führungskanal (4; 5) im Schaft (2) eines Endoskops (1) verläuft und außerhalb des Schaftes (2) mit einem Führungselement (10) auf den entsprechenden Führungskanal ausgerichtet abgestützt und geführt ist. Das Führungselement (10) nimmt proximal das auch als Handhabe dienende Gehäuse (11a) des Sondenantriebs (11) auf und ist mit seinem distalen Ende lösbar an einem mit Hähnen (6, 7) versehenen und mit dem proximalen Ende des Endoskops verbindbaren Spül- und Sondierungsaufsatz (3) lösbar befestigbar. Das Führungselement (10) ist so ausgeführt, daß die Hähne ungehindert betätigbar sind .

## Beschreibung

Die Erfindung betrifft ein Instrument für die Lithotripsie mit einer von einem Antrieb in Schwingungen versetzbaren Sonde, die durch einen Kanal im Schaft eines Endoskops verläuft und proximal außerhalb des Endoskops mit einem Führungselement auf den erwähnten Kanal ausgerichtet abgestützt und geführt ist.

Bei der endoskopischen Lithotripsie werden Ultraschallsonotroden, Pneumatiksonden, Laser oder EHL-Sonden durch den dafür vorgesehenen Kanal einer entsprechenden Operationsoptik bis zum Konkrement hin durchgeschoben. Am proximalen Ende dieser Sonden befindet sich ein in der Regel relativ schweres Handhabenteil mit dem Ultraschallwandler, dem Antriebsteil einer Pneumatiksonde oder dergleichen.

Ein Instrument der eingangs erwähnten Art ist beispielsweise aus dem DE-GM 77 05 947 bekannt. Dieses Instrument umfaßt einen Endoskopschaft, in dem eine Ultraschallsonde durch einen Ultraschallwandler in Längsrichtung bewegbar und eine Absaugeinrichtung angeordnet sind. Der Ultraschallwandler ist in einer ein Handstück bildenden Halterung befestigt, der distal ein Führungselement für die Ultraschallsonde folgt. Dieses Handstück ist gegen Federkraft auf Führungsstangen über scherenartig betätigbare Griffelemente axial verschiebbar, um die Ultraschallsonde an das zu zertrümmernde Konkrement schieben zu können. Ein Hilfsinstrument und eine Einführungshähne hierfür umfassende Anschlußeinheit sind bei diesem Instrument nicht vorgesehen.

Die Aufgabe der Erfindung besteht in der Schaffung eines Instrumentes für die Lithotripsie, welches im Vergleich zu vorbekannten Instrumenten im Aufbau einfacher und leichter ist. Weiterhin soll eine exakte Führung für starre und halbstarre Sonden gewährleistet sein. Schließlich sollen die wesentlichen Teile des Instrumentes einfach montiert und demontiert werden können.

Diese Aufgabe wird erfindungsgemäß dadurch gelöst, daß das Führungselement mit seinem distalen Ende lösbar an einem mit Hähnen versehenen, mit dem Endoskop verbundenen Spül- und Sondierungsaufsatz befestigt ist und einen von seinem distalen Ende aus proximalwärts verlaufenden Schlitz aufweist, der in eine Aussparung übergeht, durch welche der Einführungshahn für die Sonde zwecks Betätigung zugänglich ist und ein weiterer Hahn zum Einführen eines Hilfsinstrumentes nach außen ragt, und daß im Führungselement ein Kanal als Sondenführung vorgesehen ist.

Die damit erzielbaren Vorteile bestehen insbesondere in der vielseitigen Anwendbarkeit eines solchen Gerätes, das beispielsweise auch den Einsatz einer flexiblen Sonde nach Entfernen des Führungselementes zuläßt. Darüber hinaus ergibt sich eine leichte Montier- bzw. Demontierbarkeit und eine stabile, gleichwohl aber gefühlvoll betätigbare Einheit, die leicht gereinigt und sterilisiert werden kann.

Eine bevorzugte Ausgestaltung des Führungselementes sieht vor, daß in adaptiertem Zustand auch die Handhabe des Einführungshahnes für die Sonde durch die Aussparung nach außen ragt, so daß dieser Einführungshahn ungehindert betätigbar ist.

Eine kurzbauende Einheit mit relativ geringem Gewicht und günstiger Schwerpunktlage ergibt sich, wenn das Führungselement im wesentlichen zylinderförmig ist sowie proximal einen ersten Raum für die teilweise Aufnahme des Gehäuses des Sondenantriebs und distal einen zweiten Raum für die Aufnahme des Sondeneinführungshahnes aufweist und der Führungskanal für die Sonde in einer die beiden erwähnten Räume trennenden Wand vorgesehen ist.

Um bei der Montage eine einfache Einführung der Ultraschallsonden zu ermöglichen, kann der distale Endbereich des erwähnten ersten Raumes in dem Führungselement sich kegelig in Richtung auf den Führungskanal verjüngen.

Um das Instrument leicht montieren und demontieren zu können, werden das distale Ende des Führungselementes lösbar am Spül- und Sondierungsaufsatz mit einer Schraube fixiert sowie der Spül- und Sondierungsaufsatz und das Endoskop lösbar miteinander verbunden. Die hierdurch bedingte leichte Demontierbarkeit ermöglicht auch die problemlose Zerlegung zur Reinigung und Sterilisation der Instrumententeile.

Für eine griffsichere Handhabung des Instruments kann das Führungselement Zylinderform aufweisen und mindestens im Bereich des den ersten Raum umschließenden Teils als Handhabe ausgebildet sein. Außerdem bildet das Gehäuse des Antriebs ebenfalls eine Handhabe.

Das erfindungsgemäße Instrument wird nachstehend anhand eines in der Zeichnung dargestellten Ausführungsbeispiels näher erläutert. Es zeigen:
- Figur 1: ein Operationsendoskop mit Spül- und Sondierungsaufsatz und erfindungsgemäßem Führungselement,
- Figur 2: eine Seitenansicht des an einen Endoskopschaft adaptierbaren Spül- und Sondierungsaufsatzes mit dem an ihm zu befestigenden Führungselement,
- Figur 3: einen Querschnitt durch den Spül- und Sondierungsaufsatz längs der Schnittlinie A-B nach Figur 2,

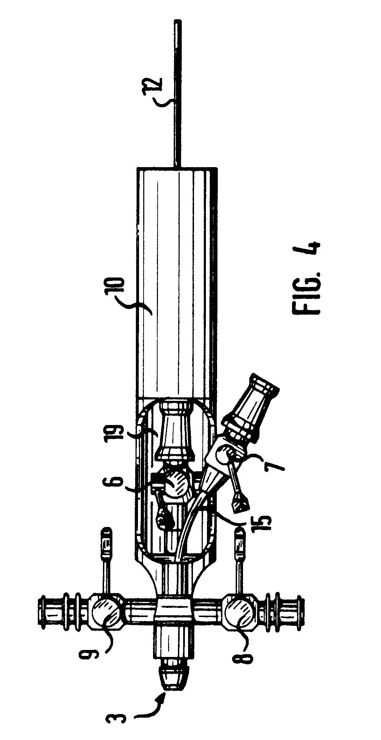

- Figur 4: eine Seitenansicht entsprechend Figur 2 in montiertem Zustand,

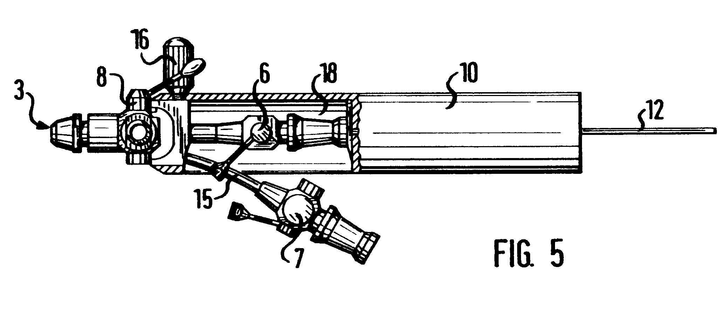

- Figur 5: eine Seitenansicht entsprechend Figur 4, aber demgegenüber um 90° gedreht und im Teilschnitt dargestellt,

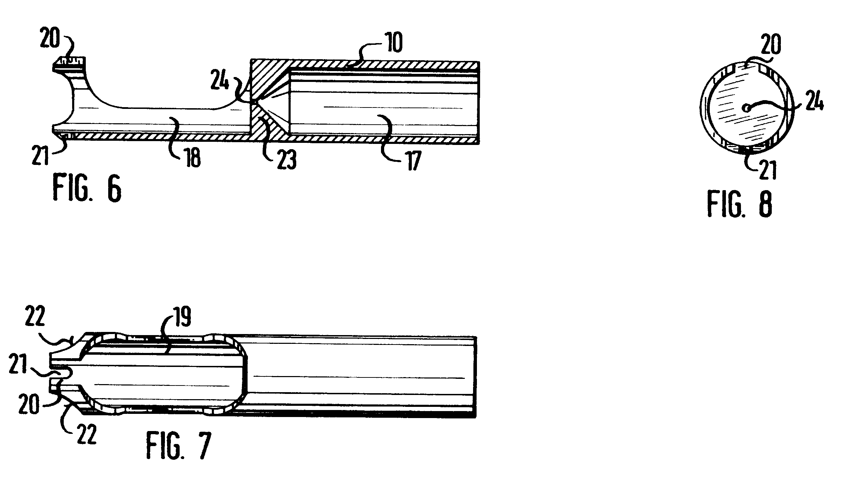

- Figur 6 und 7: einen Schnitt bzw. eine Ansicht des Führungselementes und
- Figur 8: eine Endansicht des distalen Endes des Führungselementes.

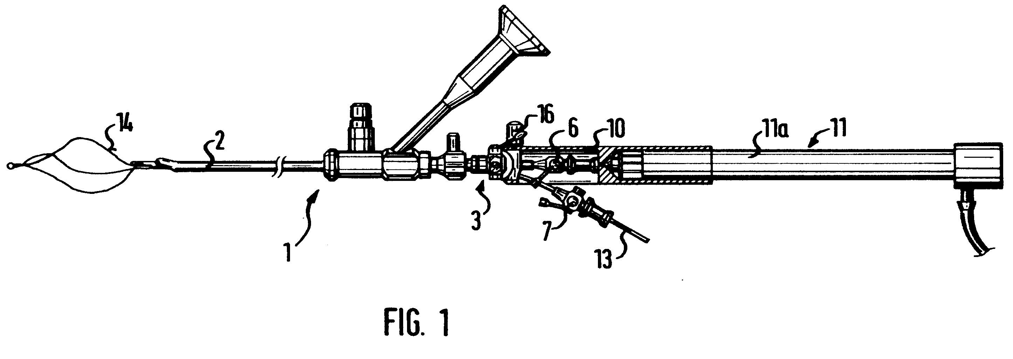

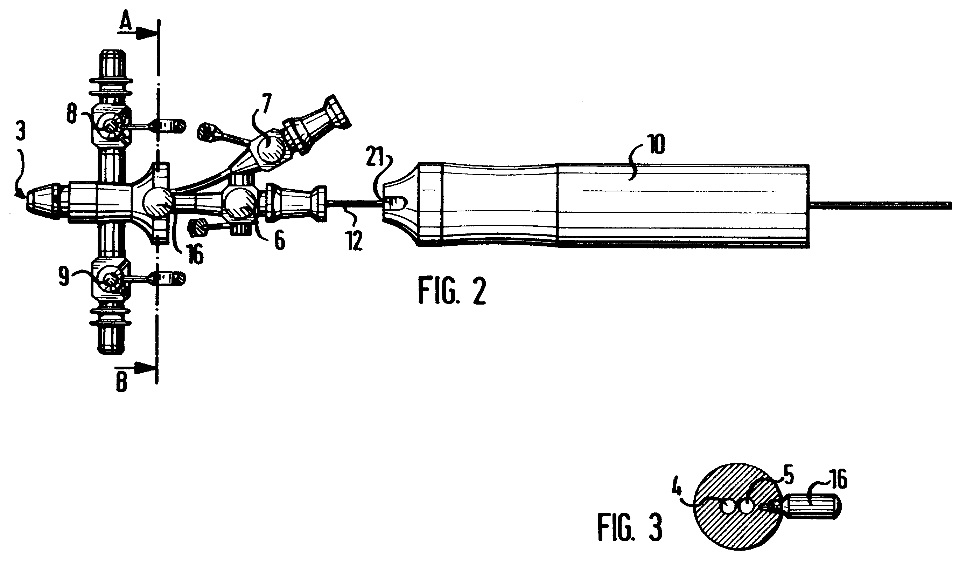

Gemäß Figur 1 besteht das für die Lithotripsie eingerichtete Instrument aus einem Endoskop 1 mit einem Schaft 2, an dessen proximalem Ende ein Spül- und Sondierungsaufsatz 3 befestigt ist. Dieser weist von mit Führungskanälen 4 und 5 (Figur 3) des Endoskopschaftes 2 in Verbindung stehende Einführhähne 6 und 7 sowie Spülhähne 8 und 9 auf. An dem Aufsatz 3 ist ein Führungselement 10 lösbar befestigt, das mit seinem distalen Ende den Aufsatz 3 teilweise übergreift und in dessen proximales Ende das zylindrische, als Handhabe dienende Gehäuse 11a eines Sondenantriebs 11 gesteckt ist. Das Führungselement führt proximal eine das gesamte Instrument bis an das distale Ende des Endoskopschaftes 2 durchlaufende Ultraschallsonde 12. Die in Figur 1 dargestellte Ausführung deutet ein Hilfsinstrument 13 mit einer Schlinge 14 zum Fixieren des zu zerstörenden Konkrementes an. Das Hilfsinstrument verläuft durch den Einführungshahn 7, ein Einführungsrohr 15 und einen der Kanäle 4, 5.

Die Festlegung des Führungselementes 10 an dem Aufsatz 3 erfolgt durch eine Klemmschraube 16, die in eine Gewindebohrung des Aufsatzes 3 geschraubt ist und das Führungselement 10 kraftschlüssig mit dem Aufsatz verbindet.

Das Führungselement 10 ist im wesentlichen zylinderförmig ausgebildet und hat proximalseitig einen ersten Raum 17 in Form einer zylindrischen Bohrung für die Aufnahme des einen Endes des ebenfalls zylindrisch ausgeführten Antriebsgehäuses 11a, wobei die Durchmesser so abgestimmt sind, daß sich ein leichter Schiebesitz ergibt. Das Führungselement 10 weist distalseitig einen zweiten Raum 18 auf, der durch eine seitliche Aussparung 19 zugänglich ist. Die Aussparung 19 ist zum distalen Ende hin durch einen Schlitz 20 als Durchlaß für das Einführungsrohr 15 beim Aufsetzen des Führungselementes 10 auf dem Aufsatz 3 geöffnet. Diesem Schlitz 20 liegt ein offenes Langloch 21 für den Gewindeschaft der Klemmschraube 16 gegenüber. Weiter ist das Führungselement 10 an seinem distalen Ende mit seitlich einander gegenüberliegenden Kehlungen 22 versehen, um einen freien Zugang zu den Betätigungshebeln der Spülhähne 8 und 9 zu gewährleisten.

Die beiden Räume 17 und 18 sind voneinander durch eine Wand 23 getrennt, in welcher ein Führungskanal 24 für die Ultraschallsonde 12 vorgesehen ist. Dabei ist der distale Endbereich der Wand 23 sich kegelig in Richtung auf den Führungskanal 24 verjüngend ausgeführt, um die Ultraschallsonde 12 beim Einführen auf den Führungskanal 24 zu lenken.

Bei der Montage wird zweckmäßigerweise so vorgegangen, daß das Führungselement 10 über den Einführhahn 6 für die Ultraschallsonde 12 geschoben wird, und zwar so ausgerichtet, daß das Einführungsrohr 15 in den Schlitz 20 eintreten kann. Hierbei wird der Gewindeschaft der Klemmschraube 16 in das Langloch 21 eingeführt, und anschließend wird die Klemmschraube angezogen. Der Einführhahn 7 für das Hilfsinstrument 13 und auch der Hebel des Einführungshahnes 6 für die Sonde 12 ragen dann seitlich aus der Aussparung 19 heraus, so daß die Einführhähne 6 und 7 ungehindert betätigbar sind. Nach Durchschieben der Ultraschallsonde 12 durch den Führungskanal 24 und den ihm zugeordneten Kanal im Endoskopschaft 2 und nach Einschieben des distalen Endes des Antriebsgehäuses 11a in den Raum 17 des Führungselementes 10 ist das Instrument betriebsbereit.

Das erfindungsgemäße Instrument ist vor allem für den Einsatz von starren oder halbstarren Sonden vorgesehen, für die eine exakte Führung erforderlich ist, um Leistungsverluste durch Reibung und Abrieb in den Sondenführungskanälen zu vermeiden. Wenn dagegen mit flexiblen Sonden gearbeitet werden soll, wird einfach das Führungselement entfernt, so daß das Operationsendoskop und der Aufsatz als solche auch für flexible Sonden zur Anwendung kommen können.

## Patentansprüche

1. Instrument für die Lithotripsie mit einer von einem Antrieb (11) in Schwingungen versetzbaren Sonde (12), die durch einen Kanal (4 oder 5) im Schaft (2) eines Endoskops (1) verläuft und proximal außerhalb des Endoskops mit einem Führungselement (10) auf den erwähnten Kanal ausgerichtet abgestützt und geführt ist, dadurch gekennzeichnet, daß das Führungselement (10) mit seinem distalen Ende lösbar an einem mit Hähnen versehenen, mit dem Endoskop (1) verbindbaren Spül- und Sondierungsaufsatz (3) befestigt ist und einen von seinem distalen Ende aus proximalwärts verlaufenden Schlitz (20) aufweist, der in eine Aussparung (19) übergeht, durch welche der Einführungshahn (6) für die Sonde (12) zwecks Betätigung zugänglich ist und ein weiterer Hahn (7) zum Einführen eines Hilfsinstrumentes (13) nach außen ragt, und daß im Führungselement (10) ein Kanal (24) als Sondenführung vorgesehen ist.

2. Instrument nach Anspruch 1, dadurch gekennzeichnet, daß die Handhabe des Einführungshahnes (6) für die Sonde (12) durch die Aussparung (19) nach außen ragt.

3. Instrument nach Anspruch 1 oder 2, dadurch gekennzeichnet, daß das Führungselement (10) im wesentlichen zylinderförmig ist sowie proximal einen ersten Raum (17) für die Aufnahme eines Endes des Antriebsgehäuses (11a) und distal einen zweiten Raum (18) für die Aufnahme des Sondeneinführungshahnes (6) aufweist und daß der Führungskanal (24) in einer die erwähnten Räume (17, 18) trennenden Wand (23) vorgesehen ist.

4. Instrument nach Anspruch 3, dadurch gekennzeichnet, daß der distale Endbereich des erwähnten ersten Raumes (17) sich kegelig in Richtung auf den Führungskanal (24) verjüngt.

5. Instrument nach einem der vorhergehenden Ansprüche, dadurch gekennzeichnet, daß das distale Ende des Führungselementes (10) am Spül- und Sondierungsaufsatz (3) mit einer Schraube (16) fixiert ist.

6. Instrument nach einem der vorhergehenden Ansprüche, dadurch gekennzeichnet, daß der Spül- und Sondierungsaufsatz (3) und das Endoskop (1) lösbar miteinander verbunden sind.

7. Instrument nach einem der vorhergehenden Ansprüche, dadurch gekennzeichnet, daß das Führungselement (10) im wesentlichen eine Zylinderform aufweist und mindestens im Bereich des den ersten Raum (17) umschließenden Teils als Handhabe ausgebildet ist und daß das Gehäuse (11a) des Antriebs (11) ebenfalls als Handhabe ausgestaltet ist.
